# EUROPEAN PATENT SPECIFICATION

(11) **EP 1 429 706 B1**
(45) Date of publication and mention of the grant of the patent: **05.12.2007**
(21) Application number: 02716474.8
(22) Date of filing: 19.01.2002
(51) Int. Cl.: A61F 13/84

(54) **INDIVIDUAL PACKING SHEET AND PACKING BAG FOR DISPOSABLE SANITARY NAPKIN**
INDIVIDUELLE VERPACKUNGSFOLIE UND VERPACKUNGSBEUTEL FÜR EINWEGMONATSBINDEN
FEUILLE D'EMBALLAGE INDIVIDUEL ET SACHET POUR SERVIETTE HYGIENIQUE JETABLE

(30) Priority: 29.09.2001 KR 2001061058
(43) Date of publication of application: 23.06.2004
(73) Proprietor: Hanjin Printing & Chemical Co., Ltd., Seoul 153-023 (KR)
(72) Inventor: SHIM, Jae-Hun, 104-708 Deoia-Apartment, Kongju-shi, Chungchangnam-do 314-110 (KR); LEE, Soo-Young, 1213-808 Haan-Apartment, Kyungmoung-shi, Kyunggi-do 423-060 (KR)
(74) Representative: GROSSE BOCKHORNI SCHUMACHER
(86) International application number: PCT/KR2002/000090
(87) International publication number: WO 2003/028609

(56) References cited:
- EP-A- 0 880 954
- EP-A- 0 955 243
- EP-A- 1 034 762
- EP-A- 1 097 878
- WO-A-00/40183
- WO-A-97/06947
- WO-A-97/12572
- WO-A-97/29723
- GB-A- 2 298 627
- KR-A- 900 017 781
- US-B1- 6 186 993
- US-B1- 6 234 229

## Description

### TECHNICAL FIELD

The present invention relates to an individual packing sheet and packing bag for a disposable sanitary napkin.

### PRIOR ART

Conventionally, a disposable sanitary napkin has a hot melt, which is coated on a reverse side of the sanitary napkin, for attaching the disposable sanitary napkin to clothes. The disposable sanitary napkin has also a release paper attached to an upper side of the hot melt, for protecting the hot melt. But, the conventional disposable sanitary napkin has a disadvantage in that a rustling sound occurs when the release paper is peeled, and it is inconvenient to peel the release paper from the disposable sanitary napkin and throw it away.

To avoid the above disadvantage and inconvenience, individually packed disposable sanitary napkins without a release paper are suggested, such as in EP 0 880 954 A1 in which silicone is coated on the inner surface of individual packing sheet of the sanitary napkin.

However, the silicone coated individual packing sheet of the sanitary napkin (hereinafter, refer to individual packing sheet) is expensive, and it is difficult to seal a packing bag made from the individual packing sheet when the sanitary napkin is packed. That is to say, one side of the individual packing sheet is wholly coated with silicone, so that thermal attaching is disturbed when the packing bag is sealed.

Meanwhile, consumers generally want the packing bag of the sanitary napkin to be soft to the touch, the rustling sound from the packing bag not to occur when the packing bag is handled, and the packing bag made from a sound-absorbing textured material.

The conventional packing bag may be cheap made from a thermosetting silicone coating agent used to manufacture a conventional release paper for protecting the hot melt, and polyethylene based film used to manufacture a packing bag of the sanitary napkin.

But, when the thermosetting silicone coating agent coated on polyethylene based film with a melting point of 100 to 130°C is dried and thermally cured at 80 to 130°C in a dry chamber with a length of 30 to 50 m, the polyethylene film becomes stiff and is deformed by heat. Furthermore, polyethylene film is deformed by heat during packing of the sanitary napkin, adhesion force of thermally sealed portion of the packing bag is weaker, as well as the demands of consumers are not met, i.e. the packing bag is not soft to the touch, and so the conventional sanitary napkin using the individual packing bag has not been successfully commercialized.

An individual packing sheet for the sanitary napkin is commercialized, one side of which is coated with a coating agent cured by an electronic or ultraviolet ray. The individual packing sheet does not deteriorate the physical properties of polyethylene film during curing of the silicone coating agent. However, a silicone coating step is complicated and production costs are high, so that competitiveness is poor-in view of cost.

In addition, an individual packing sheet of a sanitary napkin, a silicone layer of which is cured by an electronic or ultraviolet ray, has a problem in that the silicone layer is deformed by the ultraviolet ray when the individual packing sheet is exposed to the sun for a long time, as described in Japanese Patent Publication No. 1999-0082489.

Also known are package sheets, such as from EP 1,034,762 A2, which have an inner coating and an outer imprinting. EP 0,955,243 A2 or EP 1,097,878A2 teach that the inner coating can be done partially. Documents US 8,186,993 B1 and GB 2,298,627 teach how a sanitary napkin can be wrapped in a package.

### DISCLOSURE OF THE INVENTION

Therefore, it is an object of the present invention to provide an individual packing sheet for a disposable sanitary napkin coated with a low cost and high quality silicone layer, having advantages in that a production yield is increased and a production cost is reduced during a production process of the sanitary napkin by using a conventional low-priced thermosetting silicone coating agent and a packing sheet attaching a polyethylene film and a non-woven fabric with a high physical strength.

It is another object of the present invention to provide an individual packing sheet for a disposable sanitary napkin coated with a low cost and high quality silicone layer, having advantages in that the heat resistance of the film is increased by attaching a plastic film with non-woven fabric having high heat resistance, thereby thermal deformation of the film is prevented when a silicone composition coated on a plastic film is dried and thermally cured, or a hot melt is provided to the plastic film.

It is still another object of the present invention to provide an individual packing sheet for a disposable sanitary napkin, comprising a plastic film layer sandwiched between a non-woven fabric layer and a silicon layer, which is advantageous in that the non-woven fabric allows the sheet to pack the sanitary napkin under greater sealing pressures at higher sealing temperatures (by 10 to 30 °C compared to the temperature i. e. 120°C, when using conventional plastic film lacking a non-woven fabric), giving rise to an increase in the line speed, and that large allowances of thickness and uniformity are permitted in the silicon layer, thereby facilitating the thermal sealing process of the sanitary napkin with the packing sheet.

It is yet another object of the present invention to provide an individual packing sheet for a disposable sanitary napkin coated with a low cost and high quality silicone layer, having advantages in that a packing bag containing the sanitary napkin is soft to touch owing to the non-woven fabric as well as the rustling sound from the packing bag do not occur when the packing bag, made from an sound-absorbing textured material, is handled by attaching a plastic film with the non-woven fabric.

It is a further object of the present invention to provide an individual packing sheet for a disposable sanitary napkin coated with a low cost and high quality silicone layer, having advantages in that a physical strength of an individual packing sheet is increased by attaching a plastic film with a non-woven fabric having a high physical strength, thereby silicone is easily coated on the individual packing sheet and the sanitary napkin is also easily packed.

### BRIEF DESCRIPTION OF THE DRAWINGS

The above and other objects, features and other advantages of the present invention will be more clearly understood from the following detailed description taken in conjunction with the accompanying drawings, in which:
Fig. 1 schematically illustrates an attaching process of a polymer film and a non-woven fabric;
Fig. 2 schematically illustrates a coating process of a silicone coating agent on a non-woven fabric laminated polymer film.

### BEST MODES FOR CARRYING OUT THE INVENTION

According to the present invention, a plastic film does not deteriorate during a thermosetting process of a coating agent and an individual packing sheet for a disposable sanitary napkin met by consumers is provided by using conventional low-priced thermosetting silicone as a coating agent and a packing sheet attaching a polyethylene film and a non-woven fabric.

In detail, according to the present invention, a low-priced individual packing sheet for a disposable sanitary napkin and a individual packing bag made of the individual packing sheet are provided, in which the individual packing sheet for a disposable sanitary napkin comprises a non-woven fabric laminated film attaching a non-woven fabric to any one side of the polymer film of 2 to 18 g/m² with a melting temperature of 92 to 170ºC; and a silicone layer obtained by thermally curing a thermosetting silicone composition based on organopolysiloxane coated on the other side of the polymer film, to which the non-woven fabric is not contacted.

The non-woven fabric laminated film of the present invention is manufactured by laminating a thin polyethylene film of 2 to 18 g/m² on the non-woven fabric.

Although the non-woven fabric laminated film is partially cured, the present invention covers deficiencies of a cured plastic film with a soft texture of non-woven fabric by controlling a thickness of the polyethylene film thin within a range of 2 to 18 g/m².

As described above, a process for manufacturing an individual packing sheet of the present invention comprises the steps of attaching the non-woven fabric to any one side of the plastic film, coating a silicone coating composition on the other side of the plastic film, and drying a silicone coated plastic film followed by curing it.

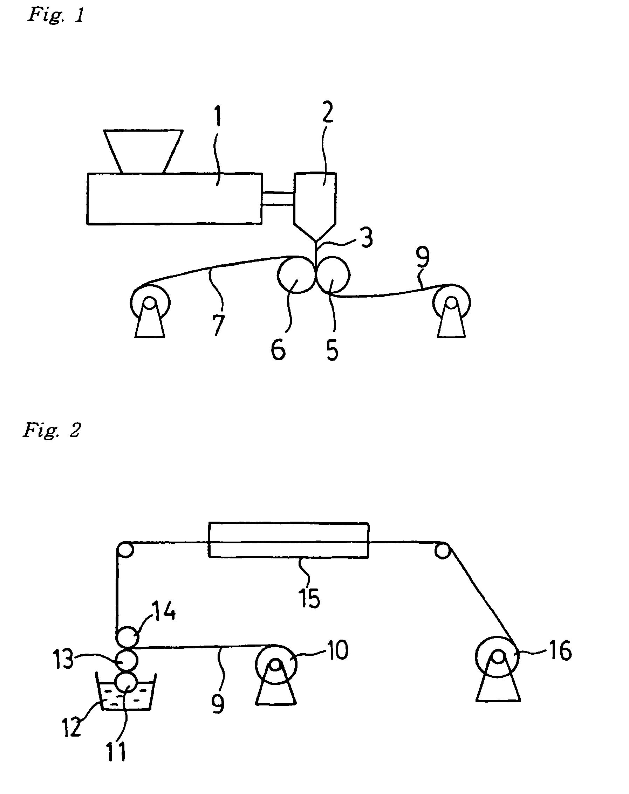

According to the present invention, the non-woven fabric is attached to the plastic film with the use of a traditional extrusion lamination line, as shown in Fig. 1.

The non-woven fiber may be selected from the group consisting of polypropylene based non-woven fabric (spun-bond non-woven fabric, thermal-bond non-woven fabric), polyester based non-woven fabric, and nylon based non-woven fabric. Polypropylene based spun-bond non-woven fabric is preferable in consideration of the price or physical properties of the individual packing sheet.

Meanwhile, it is preferable to use a polyolefin based polymer with a melting point of 92 to 170°C such as polyethylene, polypropylene in order to manufacture the plastic film. Physical properties of the plastic film are increased by combining the polyolefin based polymer with a copolymer of a polyolefin based monomer and a monomer having a polar group.

With reference to Fig. 1, there is a schematic diagram showing the extrusion and lamination of a base film. A polyethylene resin is melted at 290°C in an extruder 1 and extruded through a T-die 2 to form a film 3 of 2 to 18 g/m². The extruded film 3 is fed, along with a non-woven fabric 7, between two contacting rolls, a cooling roll 5 and a rubber mangle 6. While passing between the cooling roll 5 and the rubber mangle 6, the non-woven fabric 7 is firmly attached onto the film 3 to give a non-woven fabric laminated film 9.

The plastic film and the non-woven fabric are subjected to the extrusion lamination under a pressure applied by the cooling roll and the rubber mangle according to a traditional method. The non-woven fabric of 4 to 25 g/m² is used for the extrusion lamination.

According to the present invention, a silicone composition is coated on one side of the plastic film, to the other side of which the non-woven fabric is attached. A coating roll for coating the silicone composition may be selected from the group consisting of a direct gravure coating roll, a roll coating roll, a reverse gravure coating roll, and an offset gravure coating roll. The offset gravure coating roll is used in example of the present invention.

With reference to Fig. 2, the offset gravure coating procedure is illustrated. The non-woven fabric attached plastic film 9 from a roll 10 is provided between a coating roll 13 and a rubber mangle 14. And then, the coating composition 12 is provided to the coating roll 13 with the use of an applicator roll 11, so that a coating composition is wholly or partially coated on one side of the non-woven fabric laminated film, to the opposite side of which the non-woven fabric is attached. Next, the coating composition is dried and cured in a curing chamber 15, followed by being wound on a roll 16. To partially coat the film, a silicone coating pattern sheet copper is set on the coating roll.

An amount of the silicone coating composition is controlled so that a silicone coating layer is 0.5 to 3 g/m² after the silicone coating composition is dried. The silicone composition is cured in a curing chamber at 110ºC after the silicone composition is dried.

According to the present invention, a thermosetting silicone composition comprises main components, crosslink agents, and catalysts. Reactive organo polysiloxane such as dimethyl methylvinyl siloxane and dimethyl methyl hydrogen siloxane dissolved in an organic solvent, water, or alcohol is used as the main component.

The crosslink agent links molecules of main components with each other and causes the main component to adhere to the film. The catalyst promotes a cross-linking reaction of the crosslink agent with the main component.

Main components, crosslink agents, and catalysts are dissolved in an organic solvent, water, or alcohol, coated on the film with the use of the roll, and the solvent is volatilized.

When a mixture of main components, crosslink agents, and catalysts is sufficiently low in density to be coated on the film, the solvent is not needed. A non-solvent type silicone composition is also commercialized.

After the solvent is volatilized, the mixture of the main component, the crosslink agent, and the catalyst is cured at 60ºC or higher, and then the silicon layer allows a hot melt layer of the disposable sanitary napkin to be detachably attached thereto.

Organosiloxane such as methyl hydrogen siloxane and dimethyl hydrogen siloxane may be used as the crosslink agent. A mixture of platinum organosiloxane compound and organosiloxane is used as the catalyst.

A better understanding of the present invention may be obtained in light of the following examples which are set forth to illustrate, but are not to be construed to limit the present invention.

### EXAMPLE 1

A mixture of 98 parts by weight of linear low density polyethylene (LOPE 960, manufactured by Hanhwa General Chemiclas Co., Korea) and 2 parts by weight of TiO₂ was molded to a film of 11 g/m². A spun-bond polypropylene non-woven fabric of 15 g/m² was attached to one side of the resulting polyethylene film by extrusion lamination. Next, with the use of a coating roll, a silicon composition was coated on the other side of the polyethylene film in such an amount that the resulting three ply structure had a silicon coating layer of 0.9 g/m².

20 parts by weight of organopolysiloxane (syl-off(R)7420, manufactured by Dow Corning Co.) as the main component, 0.2 parts by weight of organosiloxane (syl-off(R)297) as the crosslink agent, 0.1 parts by weight of platinum-contained organosiloxane (syl-off(R)4000) as the catalyst, and 79.7 parts by weight of normal hexane were charged into a mixer, and then mixed for 3 hours or more to produce the silicone composition.

The silicone composition coated on the film with the use of a coating roll was dried, and cured by passing the film at a speed of 50 m/min through a dry chamber at 100°C.

Upon the extrusion lamination, the polyethylene was melted at 290°C and the film was passed at a line speed of 80 m/min under a pressure of 6 kg/cm² between the cooling roll and the rubber mangle.

Compared to a packing sheet for a disposable sanitary napkin according to the present invention, a disposable sanitary napkin was individually packed with the use of the packing sheet of the present invention so that the non-woven fabric was attached to the outside of the packing sheet and a silicone coating layer of the packing sheet contacted to the hot melt of the sanitary napkin, in a packing process of the sanitary napkin according to a conventional method in which the silicone composition coated on polyethylene film was cured by an infrared ray.

The packing bag, which is composed of the packing sheet, of the present invention was compared with the packing bag according to a conventional method, and the results are as follows:

### 1) Productivity

A heat resistance and physical properties of the packing sheet of the present invention are increased by attaching the non-woven fabric to the plastic film. Therefore, the pressure required to seal the packing bag may be higher, and the temperature required to seal the packing sheet may be higher than 120°C by 10 to 30°C in comparison with the packing bag made of the conventional plastic film without the non-woven fabric, thereby, the time for packing the sanitary napkin is reduced and productivity of the sanitary napkin production is increased.

### 2) Release property of the packing bag

When the packing bag of the present invention is compared with a conventional packing bag made by using the infrared rays in view of a release property of the packing bag, the difference between two packing bags is negligible.

### 3) Thermal deformation

A degree of thermal deformation of the packing bags according to the present invention and a conventional method using the infrared ray is observed by the naked eye. The thermal deformation of the packing sheet by a hot melt is not observed in the packing sheet of the present invention, in which the heat resistance is improved by the non-woven fabric. On the other hand, a shriveled portion owing to the thermal deformation by the hot melt is observed in the conventional packing sheet.

### 4) Appearance

The packing sheet for the sanitary napkin of the present invention is soft to the touch and friendly to the skin, looks like a high quality material owing to its sound-absorbing textured material. Also, the rustling sound from the packing sheet of the present invention does not occur when the packing sheet is handled because an outermost side of the packing sheet is the non-woven fabric. On the other hand, the conventional packing sheet for the sanitary napkin is stiff to the touch, and the rustling sound occurs from the conventional packing sheet because the outer surface of the conventional packing sheet is composed of traditional plastic film.

### INDUSTRIAL APPLICABILITY

As described above, the present invention has advantages in that product yield and cost of the sanitary napkin are improved because the non-woven fabric having a high physical strength is attached to a plastic film by using a low-priced thermosetting silicone as a coating agent and a packing sheet attaching the non-woven fabric to a polyethylene film; a heat resistance of the film is increased by attaching the non-woven fabric having the high heat resistance to the plastic film, thereby thermal deformation of the film is prevented when a silicone composition coated on the plastic film is dried and thermally cured or a hot melt is provided to the plastic film; a sealing step is quickly conducted because a sealing temperature applied to a sealing portion of the individual packing sheet when the sanitary napkin is individually packed is higher than that of a conventional individual packing sheet by 10 to 30 °C (the plastic film without the non-woven fabric is 120 °C in a sealing temperature), and a sealing step is easily accomplished because a permitted limit of an error in a thickness and a uniformity of a coating layer is broaden; the packing sheet for the sanitary napkin is soft to the touch, looks like a high quality material owing to a sound-absorbing textured material, as well as the rustling sound from the packing sheet does not occur when the packing sheet is handled because an outermost side of the packing sheet is the non-woven fabric; and a silicone coating step and a individually packing step of the sanitary napkin are easily accomplished owing to the increased physical strength of the packing sheet because the non-woven fabric having a high physical strength is attached to the plastic film.

The present invention has been described in an illustrative manner, and it is to be understood that the terminology used is intended to be in the nature of description rather than of limitation. Many modifications and variations of the present invention are possible in light of the above teachings. Therefore, it is to be understood that within the scope of the appended claims, the invention may be practiced otherwise than as specifically described.

## Claims

1. An individual, three-ply packing sheet for a disposable sanitary napkin, comprising:
a polymer film of 2 to 18 g/m² with a melting temperature of 92 to 170°C;
a non-woven fabric of 4 to 25 g/m², attached to the outer side of the polymer film; and
a silicon layer coated on the inner side of the polymer film, formed by applying a thermosetting silicon composition based on organopolysiloxane and thermally curing the silicon composition, said silicon layer allowing a hot melt layer of the disposable sanitary napkin to be detachably attached thereto.

2. An individual packing bag for a disposable sanitary napkin, made of a three-ply packing sheet comprising:
a polymer film of 2 to 18 g/m² with a melting temperature of 92 to 170°C;
a non-woven fabric of 4 to 25 g/m², attached to the outer side of the polymer film; and
a silicon layer coated on the inner side of the polymer film, formed by applying a thermosetting silicon composition based on organopolysiloxane and thermally curing the silicon composition, said silicon layer allowing a hot melt layer of the disposable sanitary napkin to be detachably attached thereto.

## Patentansprüche

1. Dreilagige Einzelverpackungsfolie für Einweg-Monatsbinde, aufweisend:
einen Polymerfilm von 2 bis 18 g/m² mit einer Schmelztemperatur von 92 bis 170°C;
ein Vliesstoffmaterial von 4 bis 25 g/m², das an der Außenseite des Polymerfilms angebracht ist; und
eine Silkonschicht, mit der die Innenseite des Polymerfilms beschichtet ist und die durch Aufbringen einer wärmehärtenden, auf Organopolysiloxan basierenden Silikonzusammensetzung und durch thermisches Aushärten der Silikonzusammensetzung ausgebildet ist, wobei die Silikonschicht gestattet, dass an ihr eine Schmelzklebstoffschicht der Einweg-Monatsbinde lösbar befestigt wird.

2. Einzelverpackungsbeutel für Einweg-Monatsbinde, der aus einer dreilagigen Einzelverpackungsfolie hergestellt ist, aufweisend:
einen Polymerfilm von 2 bis 18 g/m² mit einer Schmelztemperatur von 92 bis 170°C;
ein Vliesstoffmaterial von 4 bis 25 g/m², das an der Außenseite des Polymerfilms angebracht ist; und
eine Silkonschicht, mit der die Innenseite des Polymerfilms beschichtet ist und die durch Aufbringen einer wärmehärtenden, auf Organopolysiloxan basierenden Silikonzusammensetzung und durch thermisches Aushärten der Silikonzusammensetzung ausgebildet ist, wobei die Silikonschicht gestattet, dass an ihr eine Schmelzklebstoffschicht der Einweg-Monatsbinde lösbar befestigt wird.

## Revendications

1. Feuille d'emballage individuel à trois couches pour serviette hygiénique jetable, comprenant :
un film de polymère de 2 à 18 g/m² ayant une température de fusion de 92 à 170°C ;
un textile non tissé de 4 à 25 g/m² fixé à la face extérieure du film de polymère ; et
une couche au silicium couchée sur la face intérieure du film de polymère, formée en appliquant une composition thermodurcissable au silicium basée sur un organopolysiloxane et en durcissant thermiquement la composition au silicium, ladite couche au silicium permettant d'appliquer sur elle de manière amovible une couche thermofusible de la serviette hygiénique jetable.

2. Sachet d'emballage individuel pour serviette hygiénique jetable, constitué d'une feuille d'emballage à trois couches comprenant :
un film de polymère de 2 à 18 g/m² ayant une température de fusion de 92 à 170°C ;
un textile non tissé de 4 à 25 g/m² fixé à la face extérieure du film de polymère ; et
une couche au silicium couchée sur la face intérieure du film de polymère, formée en appliquant une composition thermodurcissable au silicium basée sur un organopolysiloxane et en durcissant thermiquement la composition au silicium, ladite couche au silicium permettant d'appliquer sur elle de manière amovible une couche thermofusible de la serviette hygiénique jetable.
